Europäisches Patentamt

European Patent Office

Office européen des brevets

(19)

(11) Numéro de publication : **0 060 776**
**B1**

(12) **FASCICULE DE BREVET EUROPÉEN**

(45) Date de publication du fascicule du brevet :
26.06.85

(21) Numéro de dépôt : 82400431.1

(22) Date de dépôt : 10.03.82

(51) Int. Cl.⁴ : **C 07 C 59/68,** A 61 K 31/19//
C07C51/09, C07C69/736,
C07C67/307, C07C79/46,
C07C76/02

(54) **Médicaments déstinés au traitement de l'obésité et des hyperlipidémies.**

(30) Priorité : 11.03.81 FR 8104834

(43) Date de publication de la demande :
22.09.82 Bulletin 82/38

(45) Mention de la délivrance du brevet :
26.06.85 Bulletin 85/26

(84) Etats contractants désignés :
BE DE FR GB IT

(56) Documents cités :
FR-A- 1 227 221
GB-A- 994 611
US-A- 3 210 413
JOURNAL OF MEDICINAL CHEMISTRY, volume 6, no.
5, septembre 1963 (US) B. BLANK et al. "Thyromimetics. I. The synthesis and hypocholesteremic activity
of some 3' and 3',5'alkyl and aryl-3,5-diiodothyroni-
nes" et "Thyromimetics. II. The synthesis and hypocholesteremic activity of some beta-diethylaminoethyl
esters of iodinated thyroalkanoic acids" pages 554-
563
CHEMICAL ABSTRACTS, volume 60, no. 6, 16 mars
1964, colonne 7328a COLUMBUS OHIO (US) C.M.
GREENBERG et al. "Relative activities of several 3'-
and 3',5'-alkyl and aryl thyromimetic agents"
CHEMICAL ABSTRACTS, volume 61, no. 10, 9 novembre 1964, colonne 12285c COLUMBUS OHIO (US)

(73) Titulaire : SOMACHIM S.A.
17, Rue Saint Florentin
F-75008 Paris (FR)

(72) Inventeur : Michel, Raymond
3 rue Charles Dickens
F-75016 Paris (FR)
Inventeur : Vendel, Jean-René
33 rue Anna Jacquin
F-92100 Boulogne (FR)
Inventeur : Bourquin, Camille
39 rue Emile Zola
F-94140 Alfortville (FR)

(74) Mandataire : Petit, Hélène et al
CABINET Pierre LOYER
18 Rue de Mogador
F-75009 PARIS (FR)

## Description

L'invention concerne des médicaments destinés au traitement de l'obésité et des hyperlipidémies. Ces médicaments comportent comme substance active un dérivé de l'acide thyroacétique, qui est l'acide 3,5-diiodo-3'-isopropyl-thyroacétique.

Certains dérivés de cet acide sont connus, notamment par les publications suivantes :

— B. Blank et al, « Thyromimetics I. The synthesis and hypocholesteremic activity of some 3' and 3',5'-alkyl and aryl-3,5-diiodo-thyronines », et « Thyromimetics II. The synthesis and hypocholesteremic activity of some β-diethylaminoethyl esters of iodinated thyroalkanoic acids », in Journal of Medicinal Chemistry, volume 6, n° 5, septembre 1963 (U.S.).

— C. M. Greenberg et al, « Relative activities of several 3'- and 3',5'-alkyl and aryl thyromimetic agents », in Chemical Abstracts volume 60, n° 6, 16 Mars 1964, colonne 7328 a, Columbus, Ohio (U.S.).

Dans ces deux publications, ce sont uniquement les propriétés hypocholesterolémiantes de dérivés de l'acide thyroacétique qui sont mises en évidence. De plus, ces publications ne font pas état, en tant que tel, de l'acide 3,5-diiodo-3'-isopropylthyroacétique. Selon l'invention, les médicaments contenant cet acide comme substance active présentent une activité intéressante dans le traitement des obésités et des hyperlipidémies.

L'invention a pour objet des médicaments pour le traitement de l'obésité et des hyperlipidémies, caractérisés en ce qu'ils comportent comme substance active l'acide 3,5-diiodo-3'-isopropyl-thyroacétique, et en ce qu'ils se présentent sous l'une des formes suivantes :

— comprimés ou gélules contenant entre 0,1 et 0,3 mg dudit acide ;
— crèmes ou laits dosés entre 0,5 et 2 mg pour mille dudit acide ;
— solutions pour ionisations dosées entre 0,1 et 1,5 mg pour mille dudit acide.

L'acide 3,5-diiodo-3'-isopropyl-thyroacétique peut être préparé par la méthode suivante :

On prépare une solution en quantités équimoléculaires d'acétate d'éthyl 4-Hydroxy-3,5-dinitrophényle et de chlorure de p-toluènesulfonyle dans la pyridine, on chauffe et remue sur un bain de vapeur pendant 15 minutes. On enlève le bain de vapeur et on ajoute un léger excès de 3-isopropyl-4-méthoxyphénol. La solution est chauffée à reflux et sous agitation pendant 2 heures, puis la pyridine est enlevée sous vide. Le résidu est dissout dans le benzène et la solution benzénique est lavée tour à tour avec de l'acide chlorhydrique dilué, de l'eau, de la soude à 10 %, et à nouveau avec de l'eau. On sèche sur du sulfate de sodium, on enlève le benzène et le résidu huileux est cristallisé et recristallisé dans l'éthanol à 95 % ; on obtient 55 % d'acétate d'éthyl [4-(3-isopropyl-4-méthoxyphénoxy)-3,5-dinitrophényle].

Point de fusion : 79-81°.

Le dinitroester ainsi obtenu est réduit catalytiquement dans un appareil de Parr en présence de palladium sur charbon actif à 10 %. Le composé diamino résultant non isolé est tétrazoté avec de l'acide nitrosyl sulfurique, et on iode le sel tétrazonium obtenu. L'ester brut diiodo est dissout dans le benzène et la solution benzénique est lavée successivement avec du bisulfite de sodium à 10 % de l'eau, du bicarbonate de sodium à 5 %, et à nouveau avec de l'eau. L'évaporation de la solution benzénique séchée donne une huile brune que l'on redissout dans un petit volume de benzène et qu'on passe à travers une colonne de Woelm acide alumine en utilisant du méthanol à 10 % dans le benzène. L'évaporation des fractions de la colonne donne une huile rougeâtre que l'on fait cristalliser dans l'éthanol aqueux pour donner 42 % de produit brut fondant entre 65 et 74°.

Les recristallisations supplémentaires dans l'éthanol ou le méthanol aqueux donnent l'acétate d'éthyl-3,5-diiodo-4-(3-isopropyl-4-méthoxyphénoxy)phényle. Point de fusion : 75-76°.

On chauffe à reflux pendant trois heures un mélange de 4 g d'acétate d'éthyl-3,5-diiodo-4-(3-isopropyl-4-méthoxyphénoxy)phényle dans 100 ml d'un mélange 1 : 1 d'acides acétique et iodhydrique. On concentre la solution à moitié de son volume original et on obtient la séparation des solides. Le mélange est dilué avec de l'eau et refroidi. Le solide filtré pèse 3,5 g et fond à 157-159°. On recristallise dans l'éthanol aqueux et on obtient 3,2 g (95 %) d'acide 3,5-diiodo-3'-isopropyl-thyroacétique.

Ce produit répond aux caractéristiques suivantes :

Formule Brute $C_{17}H_{16}I_2O_4$
Masse molaire 538,12
Point de Fusion au tube de Thiele : 160-161 °C
Pureté CCM silice Merk F 254 Support plastique
Eluant : chlorobenzène-acide acétique (85/15 v/v)
1 tache : Rf = 0,46

Le produit a également été défini par résonance magnétique nucléaire R.M.N.
Appareil Varian T60 solvant $(CD_3)_2CO$
δ en ppm/Acétone $D_5$

| δ | Protons | | | integration nombre de protons |
|---|---|---|---|---|
| 7,95 | $H_2 + H_6$ | singulet | | 2 |
| 6,76 | $H_5'$ | doublet | Jo = 8,5 Hz | ⎫ |
| 6,72 | $H_2'$ | doublet | Jm = 3 Hz | ⎬ |
| 6,30 | $H_6'$ | quadruplet | Jo = 8,5 Hz | ⎪ 5 |
| | | | Jm = 3 Hz | ⎪ |
| 6-9 | O<u>H</u> + COO<u>H</u> | bosse | Echangeable $D_2O$ | ⎭ |
| 3,7 | C<u>H</u>$_2$ | singulet | | 2 |
| 3,3 | C<u>H</u> | septet | J = 7 Hz | 1 |
| 1,2 | (C<u>H</u>$_3$)2 CH | doublet | J = 7 Hz | 6 |

Les travaux effectués selon la présente invention ont montré l'activité pharmacodynamique de ces produits sur des animaux génétiquement obèses en comparaison avec des animaux normaux.

On a notamment étudié les effets de l'acide 3,5-diiodo-3'-isopropyl-thyroacétique sur la prise de poids de la ratte génétiquement obèse, par administration au moyen d'une sonde œsophagienne et en comparaison avec des rattes normales.

Les animaux employés sont des rattes Zucker âgées de 7 à 8 semaines au début de l'expérimentation. Les animaux ont été placés en cages individuelles et recevaient ad libitum une alimentation équilibrée et vitaminée spéciale pour rats, identique pour tous les lots. La boisson était également présentée *ad libitum*. Les lots d'animaux ont été établis de telle sorte que le poids de départ soit sensiblement identique pour les témoins et les traités, chaque lot comporte 6 animaux.

On a administré une dose journalière de 17,5 µg (30n mol) par 100 g. de poids corporel pendant 4 semaines. Les poids moyens des animaux traités et non traités ont été notés chaque fin de semaine chez les témoins et chez les obèses. Les résultats sont rassemblés dans le tableau ci-dessous.

| Poids moyens | Témoins | | Obèses | |
|---|---|---|---|---|
| | non traités | traités | non traités | traités |
| Poids initial moyen | 102 g | 102 g | 135 g | 135 g |
| 7e jour | 117 | 119 | 170 | 148 |
| 14' jour | 130 | 137 | 200 | 168 |
| 21e jour | 144 | 152 | 238 | 190 |
| 28e jour | 154 | 163 | 266 | 206 |

Par ailleurs, la consommation hebdomadaire d'aliments a été notée et les résultats rassemblés dans le tableau ci-dessus.

3

0 060 776

| Consommation d'aliments | Témoins | | Obèses | |
|---|---|---|---|---|
| Consom. Initiale moyenne: | Non traités | Traités | Non traités | Traités |
| | 83 | 78 | 135 | 118 |
| 7e jour | 86 | 83 | 149 | 124 |
| 14e jour | 102 | 112 | 171 | 131 |
| 21e jour | 108 | 120 | 192 | 148 |
| 28e jour | 113 | 118 | 177 | 142 |

Enfin le tableau suivant rassemble pour chaque semaine l'observation du rapport :

$$R = \text{(consommation hebdomadaire (CH))}/\text{(gain de poids hebdomadaire (GPH))}$$

| Semaines | Rats témoins | | Rats obèses | |
|---|---|---|---|---|
| | non traités | traités | non traités | traités |
| 1 | 5,5 | 5,0 | 4,3 | 9,7 |
| 2 | 7,9 | 6,1 | 5,5 | 6,4 |
| 3 | 8,1 | 8,0 | 5,0 | 6,6 |
| 4 | 10,4 | 11,4 | 6,4 | 9,1 |

La cholestérolémie a été dosée chez les animaux dont les courbes pondérales et de consommation alimentaire ont été rapportées ci-dessus. Les résultats sont les suivants :

Rats normaux non traités 0,9-1 g par litre (2,5 mmol par l.)
Rats normaux traités (4 semaines) 0,9-1 g par litre (2,5 mmol par l.)
Rats génétiquement obèses non traités
1,7-1,8 ($\bar{X} = 1,75$) g par litre (4,51 mmol par litre)
Rats génétiquement obèses traités (4 semaines)
1,1 g par litre (2,87 mmol par litre)

De cette étude pharmacodynamique, on peut tirer les conclusions suivantes.
L'acide 3,5-diiodo-3'-isopropyl-thyroacétique à la dose de 17,5 $\mu$g pour 100 g. de poids corporel ne modifie pas significativement la croissance des rattes Zucker normales. Mais il ralentit la prise de poids des rattes Zucker génétiquement obèses.
D'une manière comparable ce produit ne modifie pas la consommation alimentaire des animaux normaux, mais réduit celle des animaux obèses.
Enfin de l'observation des résultats concernant le rapport consommation hebdomadaire sur gain de poids hebdomadaire, on peut conclure que le produit testé n'a aucun effet anorexigène chez le rat.
Enfin ce produit n'a pas d'effet sur un taux de cholestérol normal mais les animaux hypercholestérolémiques voient baisser sensiblement le taux de leur cholestérol.
On a également vérifié (méthode de double marquage à la Leucine) que l'administration de l'acide 3,5-diiodo-3'-isopropyl-thyroacétique ne modifie pas le métabolisme des protéines des animaux traités.
La toxicité de l'acide 3,5-diiodo-3'-isopropyl-thyroacétique a également été examinée sur le rat.
La $DL_{50}$ s'est avérée être de 5,4 mg par kilo, le produit fut administré en solution par sonde œsophagienne à 12 animaux.

4

L'observation pendant 14 jours de ces animaux a permis de constater qu'ils ne présentaient aucun trouble de comportement ni de croissance.

Par ailleurs, le 1/5 de la dose L 50 a été administré à 20 rats durant 30 jours, 7 jours sur 7. Aucune mort n'a été constatée. A l'autopsie aucun organe n'était macroscopiquement modifié à l'exception d'une légère pâleur des thyroïdes. L'histologie du foie, rate, rein, thyroïde, surénales n'a rien révélé d'anormal. La formule sanguine est normale. Les composantes biochimiques principales, glycémie, uricémie, urémie, évoluent dans les limites habituelles à l'espèce.

De plus, un test de Manusson et Kligman a été pratiqué chez le cobaye. Ce test prédictif s'est révélé négatif, c'est-à-dire qu'aucun animal n'a montré une quelconque sensibilisation d'ordre allergique au produit.

Il apparaît que le remplacement de l'iode par un radical isopropyl empêche la formation d'une semiquinone et exclut ainsi toute possibilité de réaction irritative ou d'ordre allergique.

Ceci constitue un avantage important du nouveau médicament.

Différents essais en clinique humaine ont été pratiqués avec l'acide 3,5-diiodo-3'-isopropyl-thyroacétique et sont les suivants.

On a administré à un groupe de 5 volontaires obèses une dose journalière de 0,3 mg d'acide 3,5-diiodo-3'-isopropylthyroacétique en 3 prises orales, durant une première période de 5 jours durant lesquels la tolérance fut vérifiée. On a surveillé la tension artérielle, le rythme cardiaque, le dosage des transaminases et phosphatases, le glucose, l'urée sanguine et la créatimine urinaire : aucun paramètre n'a subi de modification hors les variations individuelles normales.

On a également pratiqué des patchtests qui ont montré que, comme prévu, la substance n'était pas allergogène.

Après cet essai d'autres volontaires obèses ont été traités par l'acide 3,5-diiodo-3'-isopropyl-thyroacétique à la dose journalière de 0,3 mg en 2 prises orales durant 4 semaines. La tolérance fut surveillée très attentivement (malades hospitalisés) et fut parfaite.

Conformément à ce qui avait été constaté dans les expérimentations effectuées sur les rats on s'aperçoit, de façon inattendue comme chez les animaux, que l'administration du médicament selon l'invention entraîne une baisse de poids très substantielle chez l'homme obèse.

Le poids des 6 patients obèses hospitalisés sous régime hypocalorique, hypoglucidique, hyperprotidique à 1 400 cal/j et traités comme il est indiqué ci-dessus a régressé. Le tableau ci-après rassemble les résultats obtenus.

| Patient obèse | POIDS | |
|---|---|---|
| | Avant (J-0) | Après (J+30) |
| 1 | 115 | 107 |
| 2 | 108 | 101 |
| 3 | 97 | 89 |
| 4 | 103 | 93 |
| 5 | 85 | 81 |
| 6 | 111 | 104 |

De surcroît, on n'a constaté chez ces patients aucune modification des transaminases, des phosphatases, de l'uricémie et de la créatinurie. Par contre le taux de cholestérol sanguin a notablement été abaissé comme en témoigne le tableau ci-après :

(Voir Tableau page 6)

5

# 0 060 776

| Patient obèse | Taux du cholestérol sanguin | | |
|---|---|---|---|
| | Avant (J-8) | Avant (J-0) | Après (J+30) |
| 1 | 3.40 | 3.35 | 3.00 |
| 2 | 3.25 | 3.30 | 2.80 |
| 3 | 2.97 | 3.05 | 2.60 |
| 4 | 3.15 | 3.10 | 2.55 |
| 5 | 2.60 | 2.50 | 2.20 |
| 6 | 3.15 | 2.20 | 2.20 |

La glycémie de ces patients, vraisemblablement sous l'influence du régime, a diminué également de manière significative.

Le nouveau médicament selon l'invention apparaît donc utile dans le traitement de l'obésité, des hyperlipidémies et pourrait l'être dans celui des hypercholestérolémies. Il est présenté généralement sous forme de comprimés ou de gélules contenant entre 0,1 et 0,4 mg du produit actif. La posologie journalière varie entre 0,1 à 1,0 mg de produit actif répartie en 3 prises.

La formulation d'un excipient pour un comprimé du nouveau médicament est donnée ci-après à titre d'exemple :

| | |
|---|---|
| — carbonate de calcium | 19 |
| — amidon maïs | 60 |
| — talc | 20 |
| — stéarate de magnésium | 1 pour 100 g. |

Le nouveau médicament peut également être présenté sous forme de pommages, laits, crèmes ou solutions utilisables en applications locales.

La formulation d'une crème fluide pour la présentation du nouveau médicament est donnée ci-après à titre d'exemple :

| | |
|---|---|
| — cétomacrogol antoémulsionnable | 6 |
| — hexyl ester d'acide laurique | 3 |
| — huile de paraffine fluide | 0,5 |
| — paraoxybenzoate de méthyle | |
| — paraoxybenzoate de propyle | |
| — paraoxybenzoate de méthyle sodé parties égales pour 1 % | |
| — paraoxybenzoate de propyle sodé | |
| — Eau q.s. | pour 100 |

L'application journalière d'une crème dosée à 1 mg pour 1000 à 6 malades obèses a montré au bout de quatre semaines une disparition de la peau d'orange et un retour à la normale de la souplesse et de la sensibilité cutanées sur les régions du corps traitées. Cette observation permet de conclure que l'application locale du nouveau médicament lutte contre l'infiltration cellulitique du tissu conjonctif sous-cutané.

Les crèmes et lait selon l'invention sont dosés entre 0,5 et 2 mg pour mille en acide 3,5-diiodo-3'-isopropylthyroacétique.

On peut également réaliser des solutions pour ionisations dosées de préférence entre 0,1 et 1,5 mg pour mille en acide 3,5-diiodo-3'isopropyl-thyroacétique.

On peut préparer de telles solutions en dissolvant l'acide 3,5-diiodo-3'-isopropyl-thyroacétique dans un mélange :

| | |
|---|---|
| — propylène glycol | 7 |
| — eau q.s. | 93 |

et en stérilisant la solution obtenue.

**Revendication**

Médicaments pour le traitement de l'obésité et des hyperlipidémies, caractérisés en ce qu'ils comportent comme substance active l'acide 3,5-diiodo-3'-isopropyl-thyroacétique, et en ce qu'ils se présentent sous l'une des formes suivantes :
— comprimés ou gélules contenant entre 0,1 et 0,3 mg dudit acide,
— crèmes ou laits dosés entre 0,5 et 2 mg pour mille dudit acide,
— solutions pour ionisations dosées entre 0,1 et 1,5 mg pour mille dudit acide.

**Claim**

Medicaments for the treatment of obesity and hyperlipaemias, characterized in that they comprise as active substance the 3,5-diiodo-3'-isopropyl-thyro-acetic acid, and in that they present themselves in the following form :
— tablets or gelules containing from 0.1 to 0.3 mg of said acid,
— creams or milks dosed from 0.5 to 2 mg per thousand of said acid,
— ionization solutions dosed from 0.1 to 1.5 mg per thousand of said acid.

**Patentanspruch**

Arzneimittel für die Behandlung der Fettleibigkeit und der Hyperlipidämie, dadurch gekennzeichnet, daß sie als Wirkstoff 3,5-Dijod-3'-isopropylthyroessigsäure enthalten und daß sie in einer der folgenden Formen vorliegen :
— Tabletten oder Gelatinekapseln, die 0,1 bis 0,3 mg der Säure enthalten,
— Cremes oder Milche, die die Säure in einer Dosis von 0,5 bis 2 mg pro 1 000 mg enthalten,
— Lösungen für Ionisierungen, die die Säure in einer Dosis von 0,1 bis 1,5 mg pro 1 000 mg enthalten.